# EUROPEAN PATENT SPECIFICATION

(11) **EP 2 361 987 B1**
(45) Date of publication and mention of the grant of the patent: **07.08.2013**
(21) Application number: 11151429.5
(22) Date of filing: 19.01.2011
(51) Int. Cl.: C12Q 1/06, C12Q 1/02

(54) **Method for determination of reduction of micro-organisms comprised by fermenting organic material**
Verfahren zur Bestimmung der Verringerung von Mikroorganismen in einem fermentierenden organischen Material
Procédé pour la détermination de la réduction de micro-organismes par la fermentation de matière organique

(30) Priority: 18.02.2010 EP 10154014
(43) Date of publication of application: 31.08.2011
(73) Proprietor: Elsinga Beleidsplanning & Innovatie B.V., 3853 JA Ermelo (NL)
(72) Inventor: Elsinga, Willem, 3853 JA Ermelo (NL); Elsinga, Gerrit Jouke, 3853 JA Ermelo (NL); De Wit, Dirk Hendrik, 6706 ER Wageningen (NL); Duindam, Jelle Willem, 3732 AT De Bilt (NL)
(74) Representative: Van Someren, Petronella F. H. M.

(56) References cited:
- US-A- 6 146 843
- TONNER-KLANK ET AL: "Microbiological assessments of compost toilets: In situ measurements and laboratory studies on the survival of fecal microbial indicators using sentinel chambers", WASTE MANAGEMENT, ELSEVIER, NEW YORK, NY, US, vol. 27, no. 9, 1 January 2007 (2007-01-01), pages 1144-1154, XP022124677, ISSN: 0956-053X
- UDEH P J ET AL: "Field inactivation of oocysts exposed to agricultural land.", WATER AIR AND SOIL POLLUTION, vol. 142, no. 1-4, January 2003 (2003-01), pages 211-228, XP002627947, ISSN: 0049-6979
- JENKINS M B ET AL: "Use of a sentinel system for field measurements of Cryptosporidium parvum oocyst inactivation in soil and animal waste", APPLIED AND ENVIRONMENTAL MICROBIOLOGY, vol. 65, no. 5, May 1999 (1999-05), pages 1998-2005, XP002627948, ISSN: 0099-2240
- COLLICK A S ET AL: "Survival of Cryptosporidium parvum oocysts in calf housing facilities in the New York City watersheds", JOURNAL OF ENVIRONMENTAL QUALITY, vol. 35, no. 2, March 2006 (2006-03), pages 680-687, XP002627949, ISSN: 0047-2425

## Description

The present invention relates to a method for determining a reduction in the amount of micro-organisms present in fermenting organic material.

Legislation has been implemented in the EU to regulate the treatment of animal by-products in such a way that resulting products are safe and microbiological risks are eliminated. As a consequence of the implementation of this regulation, i.e. EC 1774/2002, to be replaced by EU 1069/2009 in spring 2011, it is required that organic material which comprises certain types of animal by-products is processed in particular processing facilities. For manure and/or former foodstuffs of animal origin and/or other category 3 materials as defined in the regulation, which are regarded to be such animal by-products, it is required that they are processed in only certain types of facilities. For example, EC 1774/2002 allows such material to be composted or processed in a biogas plant.

If manure and/or former foodstuffs of animal origin is composted or fermented, it must either be exposed for a minimal duration of 60 minutes to 70°C and have a maximum particle size of 12 mm, or it must be processed by a validated composting or fermentation process. As a part of the validation procedure it should be demonstrated that the process achieves a predetermined overall risk reduction.

The use of a validated procedure to allow manure and/or former foodstuffs of animal origin to be composted or fermented is an attractive alternative to the treatment for 60 minutes, 70°C and maximum particle size of 12 mm because the indicated temperature and/or time-period and maximum particle size are normally not reached in composting or fermentation installations. They can only be achieved by implementing a separate pasteurization tank or unit which needs additional investments and operational costs and result in higher energy use. On the other hand, thermophilic fermentation or composting can achieve sufficient reduction of micro organisms, even higher reductions compared with a pasteurization tank or unit.

One of the requirements which allow the use of a validated process is that the viability of certain indicator micro-organisms reduces with a predetermined amount during the composting or fermentation procedure. To establish the extent of the reduction of indicator micro-organisms, use can be made of a so-called "input-output" procedure. This rather classical procedure is based on a comparison of the number of viable indicator micro-organisms of the organic starter material with the number of the same indicator organism in the processed organic material. However, this method is not always put into practice in a straightforward manner.

For example, if the indicator micro-organism is present in the untreated organic material in relatively low concentrations, it can be technically challenging to reliably measure a sufficient reduction rate of these indicator micro-organisms after the treatment in the processed material. Also, in cases wherein the number of indicator micro-organisms is low, it has been observed that the killing-off of micro-organisms is stalled. This process could be due to the phenomenon that certain micro-organisms are present in protective niches in particles wherein they could withstand the fermentation or composting treatment.

Tonner-klank et al (2007) describe in 'Microbiological assessment of compost toilets: in situ measurements and laboratory studies on the survival of fecal microbial indicators using sentinel chambers' (Waste Management, Elsevier vol 27, no. 9 the assessment of compost toilet systems for their ability to reduce microbial indicators and pathogens. Several microbiological indicator organisms are added to sentinel chambers.

US patent 6 146 843 discloses a method and apparatus for the use of sentinel organisms. The chamber is made of a cylindrical body having a porous filter at each end.

Udeh et al (2003) describe in 'Field inactivation of oocysts exposed to agricultural land' (Water, Air and Soil Pollution, 142: 211-228) the determination of field inactivation rates of oocysts in sludge and soil, wherein oocysts were spiked into separate sentinel chambers containing 1.0 g mixture of sludge and soil.

Jenkins et al (1999) describe in 'Use of a Sentinel System for Field Measurements of Cryptosporidium parvum Oocyst Inactivation in Soil and Animal Waste' (Appl. and env. Microbiology, May 1999, p. 1998-2005) the development of small-volume sentinel chamber to asses effects of environmental stresses on survival of sucrose-Percoll-purified *Cryptosporidium parvum* oocysts in soil and animal wastes.

Collick et al (2006) describe in 'Survival of Cryptosporidium parvum Oocysts in Calf Housing Facilities in the New York City Watersheds' (in J. Environ. Qual. 35:680-687) the survivability of *C. parvum* in solar and conventional calf housing, wherein *C. parvum* oocysts were secured in sentinel chambers.

Furthermore, there are several technical complications that are inherently connected to obtaining viable counts of micro-organisms present in organic material. Existing methods for re-isolation of the target organisms may be insufficient (e.g. due to inefficient enrichment procedures for the investigated substrate) or quantification of the indicator is technically not possible due to inhibitory effects of the substrate (e.g. where certain viruses are used as indicator).

Consequently, there remains a need for the development of more efficient or alternative methods that can be used to validate composting or fermentation processes.

It is therefore an object of the present invention to provide a solution to the above-mentioned problems. This object, among others, is at least partly or completely met by the method according to the appended claims.

More in particular, this object is achieved by the provision of a method for determining the reduction in the amount of micro-organisms present in fermenting organic material
wherein biogas is produced, comprising the steps of:
a) adding one or more containers each comprising an initial predetermined number of at least one micro-organism indicator species and non-sterilized suitable organic material to fermenting organic material,
b) holding the one or more containers in the composting or fermenting organic material for a period of time,
c) determining the residual number of viable units of the at least one micro-organism indicator species,
d) calculating the reduction in viable units of the at least one micro-organism indicator species, which reduction represents the reduction in the number of viable species in the fermenting organic material wherein the one or more containers are made from a metal or a synthetic material, preferably a plastic, wherein air is squeezed out of one or more containers before air tightly closing them.

An advantage of the present invention is that the reduction in the number of viable micro-organism indicator species demonstrates the biocidal effect of a fermentation process of interest. Using the method of the invention, the rate wherein micro-organism comprised by the fermenting organic material are killed can be established on the basis of the reduction of the indicator species. The method in particular allows establishing this biocidal effect on micro-organisms which should be prevented of entering the animal feed chain or human food chain because of the health risks associated with such micro-organisms. The present invention provides a quick, reliable and more efficient method than previously known.

Furthermore, the method of the present invention makes it possible to circumvent technically challenging methods of determining micro-organismal counts in samples taken from fermenting organic material. This is especially relevant for pathogenic micro-organisms that represent a potential biological health hazard when present at low numbers in the fermenting organic material. Especially when such pathogens are highly virulent and present in numbers below the detection limit of analytic methods, it becomes increasingly difficult to detect their presence and reliably determine the reduction in their numbers. In such instances a concentration step may be required which further complicates to use of analytical methods, especially since levels of inhibiting or interfering substances may also increase. The method of the present invention makes it unnecessary to determine the amount of micro-organisms present in the fermenting organic material itself, thus providing a solution to problems related to the detection of micro-organisms present in fermenting reactors.

The method can also be used to circumvent or overcome technical problems that may be encountered when attempting to purify the micro-organisms from the fermenting material for detection thereof. Also, problems that arise from the presence of inhibiting or interfering compounds that may hamper the detection of micro-organisms can now be circumvented or overcome.

Furthermore, as the container comprises suitable organic material and as the container is surrounded, preferably in essence completely surrounded, by fermenting organic material, the indicator species are present in a similar environment as the micro-organisms comprised by the fermenting organic material to which the container is added.

Furthermore, the present invention makes practical use of the insight that sterilization of the suitable organic material is not required. Sterilization of organic material will change the environment and nutritional value for micro-organisms, thus making the conditions in the non-sterile organic material less representative for the indicator species. The present invention is thus more efficient, more representative and therefore more accurate over any method where a sterilization step is included.

In a preferred embodiment of the invention, the number of viable micro-organisms that function as indicator species initially present in the container is selected such that at least a 3 log10, or at least a 4 log10, or at least a 5 log10 reduction in the number of viable units of the micro-organism indicator species can be determined with the method of the present invention. Alternatively, the micro-organisms that function as indicator species initially present in the container, are present in a concentration of at least 10⁵ viable units/ml, or at least 10⁶ viable units/ml, or at least 10⁷ viable units/ml, or at least 10⁸ viable units/ml. Depending on the prevailing legislation or other (scientific) interests, the need to determine the extent of the reduction of indicator species may change and determine the number of initially present viable units of indicator species. One of the advantages of the present invention is that the present method provides great flexibility in the selection of unit counts of indicator micro-organisms in practicing the present method. Especially when high unit loads are used is it possible to determine the extent of the biocidal effect of fermentation processes or installations.

The term "units" as used herein means individual cells when referring to bacteria, fungi or yeasts, and individual virus particles when viruses are referred to or eggs of ascaris species or nematodes or individual seeds of weeds or tomato seeds.

The amounts of indicator organisms can be determined with methods that allow the number of colony-forming units (CFU) or plaque-forming units (PFU) per ml or gram to the established. Such methods for establishing the degree of contamination are known in the art and can easily be performed by the skilled person. For example, when determining the number of colony-forming units of bacteria, the skilled person can make serial dilutions of the contents of the container at any relevant time-point, allow the bacteria to grow and simply count the number of colonies to establish CFU counts which represents the amount of viable indicator micro-organism at the selected time-point. For determining viral loads, similar titration methods involving diluting and culturing, and possibly staining of viruses, are available to determine the number of plaque-forming units. In theory though, any other method that allows to differentiate between viable and dead micro-organisms is suitable to practice the invention.

According to a preferred embodiment of the present invention, the micro-organism that functions as indicator is a bacterium, virus, in particular a parvovirus, fungus or yeast or eggs of ascaris species or nematodes or individual seeds of weeds or tomato seeds.

More preferably, the micro-organism to be used as indicator species is a bacterium that belongs to the Enterobacteriaceae or Enterococcaceae.

Even more preferably, the bacteria used as indicator species belong to the Enterococcus genus, in particular E. *faecalis*; or belong to the Escherichia genus, in particular E. *coli*, or belong to the Salmonella genus, in particular *Salmonella* serovar Senftenberg 775W, H₂S negative, or a combination thereof.

In a preferred embodiment, the non-sterilized suitable organic material comprised by the one or more containers is the same material as the fermenting organic material to which the one or more containers are added in step a).

With the term "the same material" herein is meant that the non-sterilized organic material comprised by the one or more containers is a sample taken from the fermenting organic material to which the one or more containers are added in step a) and/or a sample taken from organic material which organic material subsequently becomes the fermenting organic material to which the one or more containers are added in step a). The latter option applies to the situation wherein organic material is supplied to a fermenting installation with the purpose of being fermented and wherefrom, prior to its addition thereof to the respective installation, a sample is taken of which a part is then added to the container. Next, the container comprising organic material from this sample is added to the same organic material which is fermenting as meant in step b). Thus in this preferred embodiment, the source of the material comprised by the container is the material to which the container is added in step a). Consequently, these organic materials are also in the same stage of the fermentation process.

By using the same organic material which is comprised by the one or more containers and to which the one or more containers are added allows the conditions in the container in essence to fully resemble the conditions to which micro-organisms comprised by the fermenting organic material are subjected. Advantageously, when compliance to EC 1774/2002 is investigated, using a sample from the organic material comprised by the fermentation facility to be investigated, allows the indicator micro-organisms to be exposed to the same biocidal activity or effects as the micro-organisms comprised by the fermenting organic material to which the container is added. The method of the present invention thus in particular allows to investigate in an efficient manner whether a fermentation facility functions such that it complies with regulation EC 1774/2002.

In a preferred embodiment, the amount of added fermenting organic material, comprised by an individual container is at least 10, 20, 30, 40, 50, 60, 70, 80, 90%, 95%, 99% or 99,9% (v/v) of the total material added to the container. In a preferred embodiment, the remaining volume of material added to the container, in particular at least 0, 1, 1, 5, 10, 20, 30, 40, 50, 60, 70, 80 or 90% (v/v) of the total material added to the container, is comprised by the medium that comprises the indicator micro-organisms.

The organic material according to the present invention preferably comprises vegetable waste, fruit waste, garden waste, manure, animal by-products, catering waste, kitchen waste other organic waste products, category 2 or category 3 waste as meant in EU Regulation 1774/2002 and EU Regulation 1069/2009, food waste or any combination thereof.

In a preferred embodiment, the organic material is thermophilically or mesophyilically fermented.

According to the present invention, the period of time of step b) comprises a time sufficient to kill-off a measurable amount of indicator micro-organisms. Such a time period can be a predetermined time when the method of the invention is used for periodical validation purposes to show that the installation still performs as expected and roughly shows the expected biocidal effect. This is the case when the method is repeatedly applied in the same fermenting installation, or in a fermenting installation of a similar type, and the results do not vary a lot. Alternatively, this period of time can be selected from at least one hour, preferably at least two hours, more preferably at least four hours, even more preferably at least 6 or 8 hours. Longer time periods are also possible, even for the complete duration of the fermentation process if the time needed to obtain results is not pressing. Preferably though, this period of time is at least one hour, preferably at least two hours, more preferably at least four hours, even more preferably at least six or eight hours for a fermentation process.

In another preferred embodiment, the more than one containers are held in the fermenting organic material for different periods of time in step b). When a plurality of such containers is used, it is possible to determine the rate with which the indicator micro-organisms are killed-off. This in turn allows to calculate, for example using linear regression, the time needed to reduce the concentration of viable indicator micro-organism by 1 log unit. If this time is known, it can be calculated what time is required to achieve a certain concentration decrease in viable indicator micro-organisms and thus the corresponding micro-organism comprised by the fermenting material to which the container is added.

The container used in the method of the present invention is provided with a closeable opening to allow addition of material therein. The closeable opening can be airtightly closed using closing means, such as, but not limited to, a lid, screw-lid, cap, screw-cap, closeable seal, and the like. It lies well within the capabilities of the skilled person to select a container comprising an opening that can be airtightly closed with suitable closing means.

According to the invention, the container is made from a metal or a synthetic material, preferably a plastic or a sterilizable plastic. The container may be a pot or bag or have any other suitable shape. Such material allows the indicator micro-organisms in the container to be subjected to the same temperature as the micro-organisms comprised by the fermenting or composting organic material when the one or more containers are placed inside the fermenting or composting organic material. Such material also allows sufficient flexibility to squeeze air out of the container before airtightly closing it such that the container can sufficiently expand from the produced biogas. This is preferred when the container is added to fermenting material because of the production of biogas.

In another preferred embodiment, a datalogger is included in the one or more containers to allow recording of at least the temperature inside the one or more containers.

In yet another preferred embodiment, the one or more containers are airtightly closed with the exception of a part which is gas-permeable but impermeable to the micro-organism. It lies well within the capabilities of the skilled person to determine which pore-size is to be selected when a certain micro-organism is used. Such a gas permeable part is preferably constituted by a filter or membrane having a pore size lying in the range of 0,01 µm to 0,45 µm, preferably 0,2 µm. In case a virus is used as indicator species, a membrane or filter can be used that falls within the ultrafilter window is preferably used. For bacteria, a membrane or filter can be used that falls within the microfiltration window.

The option of using a container having a gas permeable part is in particular of interest in a composting reactor or composting process. This allows the gas exchange to occur between the contents of the container and the composting material. This allows the indicator species to be subjected to the same aerobic conditions as to which the micro-organism comprised by the composting reactor to which the container is added are subjected.

In yet another preferred embodiment, the one or more containers are provided with a suitable protective casing. Such a casing protects the one or more containers from being pressed together, especially when applied in composting processes. Preferably, the casing is made of a sufficiently strong metal (i.e. iron or steel) or a strong plastic material (i.e. PVC). This casing will be in direct contact with the fermenting organic material. Also, the protective casing comprises holes of sufficient size to allow the regime to which the fermenting organic material is subjected to be transferred to the contents of the containers. This allows the conditions, in particular the temperature, in the container to resemble as much as possible the conditions to which the micro-organisms are subjected that are comprised by the fermenting of organic material to which the container is added. Suitably, the protective casing is an elongated hollow body, such as a tube or pipe having a rounded or rectangular cross-section perpendicular to the length axis, with a sufficient width such that one or more of the containers fit in the protective casing and remain on top of each other. It has been shown by the applicant that such a suitable protective casing still allows the indicator micro-organisms to be killed-off.

In yet another preferred embodiment, the one or more containers are attached to a line or chain to allow easy recovery thereof from the fermenting organic material.

Next, the invention is illustrated by the following non-limiting examples.

### EXAMPLES

### Example 1

Use of indicator micro-organisms in a fermentation installation.

The fermenting installation comprises two stirred reactors of 2100 m³. The organic material is mesophylically fermented for about 40 days in one reactor which is held at about 40°C and subsequently thermophylically fermented for again about 40 days in the other reactor which is held at 50-55°C.

The fermentation tanks are fed with cow manure, vegetable glycerin, vegetable oils, maize, and category three material as meant in EC 1774/2002. This material comprises unpacked foodstuffs from the supermarket which were not sold before the ultimate sell-by date. The reactor comprised at least 50% manure.

A one-liter pure *Enteroccus faecalis* culture of 3.8 10⁸ CFU was obtained and mixed with 9 liter fermenting organic material of 52.2°C taken from the thermophilic reactor. The organic material and E. *faecalis* culture were mixed for 1 minute to obtain a 10-liter stock solution comprising a predetermined amount of E. *faecalis* CFU of about 10⁷ (i.e. a solution comprising 7 log units E. *faecalis*). One liter of this mixture was put in plastic containers (containers 1-9).

Half of the contents of these containers were transferred to a second container (reference containers 1A-9A) such that the contents of container 1 corresponded to the contents of container 1A, etc.

Containers 1A-9A were immediately cooled in ice-containing water and transferred to the refrigerator (4°C) after they were sufficiently cooled down. These containers were used to determine the precise concentration of the initial E. *faecalis* CFU. The remaining containers were attached to a chain.

The time between the mixing of the organic material with pure culture until the containers were placed in the fermentation reactor was 24 minutes. The reference containers were placed in the ice-water 8 minutes after the mixtures were prepared.

The time when the containers 1A-9A were placed in the ice water was set as t=0. For the following three hours, each half hour one container was retrieved from the reactor. The remaining containers were retrieved from the reactor at a hour time interval. Immediately after retrieval, the containers were placed in ice-water and placed in a refrigerator at 4°C.

Counts of E. *faecalis* CFU were determined after culturing serial dilutions on Slanetz and Bartley agar and incubation at 44°C for 44-48 hours.

### Results

Resulting CFU counts were obtained as described above and listed in table 1.

**Table 1. CFU counts of Enterococcus faecalis used as indicator species to assess the reduction in the numbers thereof in fermenting organic material and a fermentation installation. Cont. nr. = container number.**

| cont. | time | CFU/g | log | cont. | time | CFU/g | log | reduction |
|---|---|---|---|---|---|---|---|---|
| nr. | (min) | | CFU/g | nr. | (min) | | CFU/g | |
| 1A | 0 | 3.8 10⁷ | 7.6 | 1 | 30 | >3.4 10⁶ | >6.5 | <1.0 |
| 2A | 0 | 4.6 10⁷ | 7.7 | 2 | 60 | >3.4 10⁶ | >6.5 | <1.1 |
| 3A | 0 | 2.7 10⁷ | 7.4 | 3 | 90 | 1.8 10⁵ | 2.2 | 2.2 |
| 4A | 0 | 4.9 10⁷ | 7.7 | 4 | 120 | <10 | <1 | >6.7 |
| 5A | 0 | 4.2 10⁷ | 7.6 | 5 | 150 | <10 | <1 | >6.6 |
| 6A | 0 | 2.0 10⁷ | 7.3 | 6 | 180 | <10 | <1 | >6.3 |
| 7A | 0 | 3.8 10⁷ | 7.6 | 7 | 240 | <10 | <1 | >6.6 |
| 8A | 0 | 3.6 10⁷ | 7.6 | 8 | 300 | <10 | <1 | >6.6 |
| 9A | 0 | 3.4 10⁷ | 7.5 | 9 | 360 | <10 | <1 | >6.5 |

As mentioned, containers labeled with "A" served as reference containers to determine the initial CFU counts of the indicator species comprised by the container which is contacted with and held in the fermenting organic material. The log units of the reference containers were fairly equal and lied between 7.3 and 7.7. The concentration of the starter E. *faecalis* culture was 8.6 log units per ml. The log units of around 7.5 of the reference containers indicates that the preparation of the 10% dilution of the starter culture did not result in significant loss of viable indicator cells.

Furthermore, the results listed in table 1 demonstrate that it is possible to measure the biocidal effect of the fermentation reaction with the method according to the invention within a time-frame of already two hours. Already after 120 minutes, a reduction of 6.7 log units was demonstrated. When compliance with EC 1774/2002 is investigated, it suffices to demonstrate that a fermentation reaction has a biocidal effect resulting in a 5 log units reduction. The present invention thus allows demonstrating this effect. The method thus provides sufficient flexibility and capacity to measure the biocidal effects of a fermentation process.

### Example 2

A second fermentation installation was investigated with respect to the biocidal effects of the fermentation processes that could be measured in the installation. The reactors of the installation were continuously stirred and fed with 25% cow manure, 25% pig manure, 1% chicken manure, 14% cow manure, 20% maize, 10% vegetable glycerin, 5% unpacked foodstuffs from supermarkets which were not sold before the ultimate sell-by date 1.5% orange peels and 1% other, such as cereals. The installation comprised two parallel operated mesophylic reactors having a capacity of 1800 m³ in which the organic material is kept on average for 45 days at 39°C and one serially operated thermophilic reactor of 3300m³ capacity in which the organic material is kept on average for 41 days at 54-55°C.

Similar results as described in example 1 were obtained in the thermophilic reactor where it was shown that after about one hour, a 5.2 log reduction in the indicator organism was measured. After 90 minutes, a 5.5 log reduction was measured. In this experiment, it was demonstrated using the present invention that the installation was operated in compliance with EC 1774/2002.

### Example 3

In a third experiment, yet again performed in a comparable manner as described for example 1, it was demonstrated that after 4, 5 and 6 hours, a reduction in the amount of *Enterococcus faecalis* indicator species was measured of more than 6 log units at each of these time points. Thus, the fermentation reactions in this third installation were found to comply with EC 1774/2002 as the required reduction of 5 log units indicator species was reached within about 4 hours, whereas the average retention time of the organic material in this thermophylically operated installation was 14 days.

This installation makes use of a plug-flow reactor which is fed with sieved organic material having particle sizes of less than 80 mm and horizontally mixed. This organic material is kept in the fermentor for 14 days at about 55°C. The installation is primarily fed with source-separated municipal kitchen and garden waste and catering waste.

### Example 4

Use of indicator micro-organisms in a composting installation.

A composting process operated at 55°C by aerating the composting organic material with air of 65°C to investigate the method as mentioned under Example 1. The background CFU count *Enterococcus faecalis* indicator in the composting material was rather low at 4700 CFU/g material.

In a first experiment, a reduction of 5.7 log units of the E. *faecalis* indicator species was recorded within a time-frame of 8 hours. However, in this experiment only two containers were analyzed that were held in the composting organic material for 8 hours and 72 hours, respectively. Both containers showed a reduction of about 5.7 log units. The containers comprised an initial number of 6 log units indicator CFU/g. The composting process in the installation thus functioned in accordance to EC 1774/2002. These containers were not enclosed in a steel protective casing.

In a second experiment, the containers were placed inside a protective casing which was a steel pipe of about 1.5 meter length with a rectangular shape and holes to allow the containers to be subjected to the same temperature of the compositing material and to the same environmental conditions as the composting material, such as the process air.

In this second experiment performed in a composting installation, the biocidal effect of the compositing reactions was confirmed. After 8 hours a reduction of 5.6 log units E. *faecalis* was determined. After 24 and 96 hours, similar results were obtained.

The temperature in the containers was recorded with dataloggers. There was no apparent influence of the measured temperatures on the biocidal effects of the composting processes on the indicator species as comparable results were obtained with containers that recorded a temperature of about 55°C and about 65°C.

## Claims

1. Method for the determination of the reduction in the number of viable units of micro-organisms present in fermenting organic material wherein biogas is produced, comprising the steps of:
a) adding one or more containers each comprising an initial predetermined number of at least one micro-organism indicator species and non-sterilized suitable organic material to the fermenting organic material,
b) holding the one or more containers in the fermenting organic material for a period of time,
c) determining the residual number of viable units of the at least one micro-organism species present in the one or more containers,
d) calculating the reduction in viable units of the at least one micro-organism species to determine the reduction in the number of viable species in the fermenting organic material,
wherein the one or more containers are made from a metal or a synthetic material, preferably a plastic, wherein air is squeezed out of the one or more containers before air tightly closing them.

2. Method according to claim 1, wherein the one or more containers are provided with a protective casing which is a tube or a pipe having a rounded or rectangular cross-section perpendicular to the length axis, with a sufficient width such that the one or more containers fit in the protective casing and remain on top of each other.

3. Method according to claim 1 or 2, wherein the non-sterilized suitable organic material comprised by the one or more containers is the same material as the fermenting organic material to which the one or more containers are added in step a).

4. Method according to any of the claims 1-3, wherein the non-sterilized suitable organic material comprised by the one or more containers is a sample from the fermenting organic material to which the container is added in step a) and/or a sample from organic material which organic material subsequently becomes fermenting organic material to which the one or more containers are added in step a).

5. Method according to any of the claims 1-4, wherein the micro-organism is a bacterium, virus, fungus, yeast, eggs of ascaris species, weeds, tomato seeds, nematodes or the like.

6. Method according to any of the claims 1-5, wherein the micro-organisms is a bacterium that belongs to the Enterobacteriaceae or Enterococcaceae.

7. Method according to claim 5 or claim 6, wherein the bacteria belong to the Enterococcus genus, in particular *E. faecalis;* or belong to the Escherichia genus, in particular *E. coli,* or belong to the Salmonella genus, in particular serovar Senftenberg 775W, H₂S negative, or a combination thereof.

8. Method according to any of the claims 1-7, wherein the micro-organism is a virus, in particular a thermo-resistant virus, such as a parvovirus.

9. Method according to any of the claims 1-8, wherein the one or more containers are held in the fermenting organic material for different periods of time in step b) .

10. Method according to any of the claims 1-9, wherein the organic material comprises vegetable waste, fruit waste, garden waste, manure, animal by-products, catering waste, kitchen waste, other organic waste products, category 2 or category 3 waste as meant in EU Regulation 1774/2002 or EU Regulation 1069/2009, food waste or any combination thereof.

11. Method according to any of the claims 1-10, wherein the organic material is thermophilically or mesophilically fermented.

12. Method according to any of the claims 1 to 11, wherein the period of step b) is from 1 to 8 hours, preferably from 2 to 8 hours.

## Patentansprüche

1. Verfahren zur Bestimmung der Verringerung bei der Anzahl an lebensfähigen Einheiten von Mikroorganismen, die in fermentierendem organischem Material vorliegen, in welchem Biogas erzeugt wird, das die folgenden Schritte umfasst:
a) das Zugeben von einem oder mehreren Behältern, die jeweils eine anfängliche vorher bestimmte Anzahl von wenigstens einer Mikroorganismen-Indikatorspezies und nicht sterilisiertes geeignetes organisches Material umfassen, zu dem fermentierenden organischen Material,
b) das Halten des einen oder der mehreren Behälter für einen Zeitraum in dem fermentierenden organischen Material,
c) das Bestimmen der restlichen Anzahl an lebensfähigen Einheiten der wenigstens einen Mikroorganismenspezies, die in dem einem oder den mehreren Behältern vorliegt,
d) das Berechnen der Verringerung bei den lebensfähigen Einheiten der wenigstens einen Mikroorganismenspezies, um die Verringerung bei der Anzahl an lebensfähigen Spezies in dem fermentierendem organischen Material zu bestimmen,
wobei der eine oder die mehreren Behälter aus einem Metall oder einem synthetischen Material, vorzugsweise einem Kunststoff, gefertigt sind, wobei Luft aus dem einen oder den mehreren Behältern herausgepresst wird, bevor diese luftdicht verschlossen werden.

2. Verfahren gemäß Anspruch 1, wobei der eine oder die mehreren Behälter mit einer Schutzhülle versehen sind, welche ein Schlauch oder eine Röhre mit einem gerundeten oder rechteckigen Querschnitt senkrecht zu der Längsachse mit einer hinreichenden Breite ist, so dass der eine oder die mehreren Behälter in die Schutzhülle hineinpassen und jeweils übereinander bleiben.

3. Verfahren gemäß Anspruch 1 oder 2, wobei das nicht sterilisierte geeignete organische Material, das in dem einen oder den mehreren Behältern enthalten ist, dasselbe Material wie das fermentierende organische Material ist, zu welchem der eine oder die mehreren Behälter in Schritt a) zugegeben werden.

4. Verfahren gemäß irgendeinem der Ansprüche 1 - 3, wobei das nicht sterilisierte geeignete organische Material, das in dem einen oder den mehreren Behältern enthalten ist, eine Probe von dem fermentierenden organischen Material ist, zu welchem der Behälter in Schritt a) zugegeben wird, und/oder eine Probe von organischem Material ist, wobei das organische Material anschließend fermentierendes organisches Material wird, zu welchem der eine oder die mehreren Behälter in Schritt a) zugegeben werden.

5. Verfahren gemäß irgendeinem der Ansprüche 1 - 4, wobei der Mikroorganismus ein Bakterium, ein Virus, ein Pilz, eine Hefe, Eier von Ascaris-Spezies, Unkräuter, Tomatensamen, Nematoden oder dergleichen sind.

6. Verfahren gemäß irgendeinem der Ansprüche 1 - 5, wobei der Mikroorganismus ein Bakterium ist, das zu den Enterobacteriaceae oder Enterococcaceae gehört.

7. Verfahren gemäß Anspruch 5 oder Anspruch 6, wobei die Bakterien zu der Gattung Enterococcus, insbesondere *E. faecalis,* gehören, oder zu der Gattung Escherichia, insbesondere *E. coli,* gehören, oder zu der Gattung Salmonella, insbesondere Serovar Senftenberg 775W, H₂S negativ, gehören, oder zu einer Kombination von diesen.

8. Verfahren gemäß irgendeinem der Ansprüche 1 - 7, wobei der Mikroorganismus ein Virus, insbesondere ein wärmeresistentes Virus, wie z.B. ein Parvovirus ist.

9. Verfahren gemäß irgendeinem der Ansprüche 1 - 8, wobei der eine oder die mehreren Behälter in Schritt b) für verschiedene Zeiträume in dem fermentierenden organischen Material gehalten werden.

10. Verfahren gemäß irgendeinem der Ansprüche 1 - 9, wobei das organische Material pflanzlichen Abfall, Früchteabfall, Gartenabfall, Dung, tierische Nebenprodukte, Gaststättenabfall, Küchenabfall, andere organische Abfallprodukte, Abfall der Kategorie 2 oder Kategorie 3 nach der EU Vorschrift 1774/2002 oder der EU Vorschrift 1069/2009, Essensabfall oder irgendeine Kombination von diesen umfasst.

11. Verfahren gemäß irgendeinem der Ansprüche 1 - 10, wobei das organische Material thermophil oder mesophil fermentiert wird.

12. Verfahren gemäß irgendeinem der Ansprüche 1 bis 11, wobei der Zeitraum von Schritt b) von 1 bis 8 Stunden, vorzugsweise von 2 bis 8 Stunden, beträgt.

## Revendications

1. Procédé de détermination de la réduction du nombre d'unités viables de micro-organismes présents dans une matière organique en fermentation, où un gaz biologique est produit, comprenant les étapes consistant à :
a) ajouter un ou plusieurs récipients comprenant chacun un nombre initial prédéterminé d'au moins une espèce indicatrice de micro-organisme et une matière organique appropriée non stérilisée à la matière organique en fermentation,
b) maintenir les un ou plusieurs récipients dans la matière organique en fermentation pendant une période de temps,
c) déterminer le nombre résiduel d'unités viables de la au moins une espèce de micro-organisme présente dans les un ou plusieurs récipients,
d) calculer la réduction des unités viables de la au moins une espèce de micro-organisme afin de déterminer la réduction du nombre d'espèces viables dans la matière organique en fermentation,
où les un ou plusieurs récipients sont fabriqués à partir d'un métal ou d'un matériau synthétique, de préférence du plastique, où l'air est expulsé des un ou plusieurs récipients avant de le/les refermer hermétiquement.

2. Procédé selon la revendication 1, dans lequel les un ou plusieurs récipients sont fournis avec une enveloppe de protection qui est un tube ou un tuyau ayant une section transversale ronde ou rectangulaire qui est perpendiculaire à l'axe de la longueur, avec une largeur suffisante pour que les un ou plusieurs récipients soient placés dans l'enveloppe de protection et restent les uns sur les autres.

3. Procédé selon la revendication 1 ou 2, dans lequel la matière organique appropriée non stérilisée contenue par les un ou plusieurs récipients est la même matière que la matière organique en fermentation à laquelle les un ou plusieurs récipients sont ajoutés à l'étape a).

4. Procédé selon l'une quelconque des revendications 1-3, dans lequel la matière organique appropriée non stérilisée contenue par les un ou plusieurs récipients est un échantillon de la matière organique en fermentation à laquelle le récipient est ajouté à l'étape a) et/ou un échantillon d'une matière organique, laquelle matière organique devient ensuite une matière organique en fermentation à laquelle les un ou plusieurs récipients sont ajoutés à l'étape a).

5. Procédé selon l'une quelconque des revendications 1-4, dans lequel le micro-organisme est une bactérie, un virus, un champignon, une levure, des oeufs d'une espèce d'ascaris, des mauvaises herbes, des graines de tomates, des nématodes ou analogues.

6. Procédé selon l'une quelconque des revendications 1-5, dans lequel les micro-organismes sont une bactérie qui appartient aux Enterobacteriaceae ou aux Enterococcaceae.

7. Procédé selon la revendication 5 ou la revendication 6, dans lequel les bactéries appartiennent au genre Enterococcus, en particulier *E. faecalis* ; ou appartiennent au genre Escherichia, en particulier *E. coli,* ou appartiennent au genre Salmonella, en particulier le sérovar Senftenberg 775W, H₂S négatif, ou une combinaison de celles-ci.

8. Procédé selon l'une quelconque des revendications 1-7, dans lequel le micro-organisme est un virus, en particulier un virus thermorésistant, comme un parvovirus.

9. Procédé selon l'une quelconque des revendications 1-8, dans lequel les un ou plusieurs récipients sont maintenus dans la matière organique en fermentation pendant différentes périodes de temps à l'étape b).

10. Procédé selon l'une quelconque des revendications 1-9, dans lequel la matière organique comprend des déchets végétaux, des déchets de fruits, des déchets de jardin, du fumier, des sous-produits animaux, des déchets de restauration, des déchets de cuisine, d'autres produits de déchets organiques, des déchets de catégorie 2 ou catégorie 3, tel que défini dans la réglementation UE 1774/2002 ou dans la réglementation UE 1069/2009, des déchets alimentaires ou une quelconque combinaison de ceux-ci.

11. Procédé selon l'une quelconque des revendications 1-10, dans lequel la matière organique est soumise à une fermentation thermophile ou mésophile.

12. Procédé selon l'une quelconque des revendications 1 à 11, dans lequel la période de l'étape b) est comprise entre 1 et 8 heures, de préférence entre 2 et 8 heures.
